# EUROPEAN PATENT APPLICATION

(11) **EP 4 230 255 A1**
(43) Date of publication of application: **23.08.2023**
(21) Application number: 20958769.0
(22) Date of filing: 22.10.2020
(51) Int. Cl.: A61N 1/06, A61N 1/05, A61N 1/40, A61N 1/08, A61N 1/36, A61N 1/32

(54) **FRACTIONAL MICRONEEDLE HYBRID SWITCH MODULE MANUFACTURING METHOD**

(30) Priority: 19.10.2020 KR 20200135251
(71) Applicant: Agnes Medical Co., Ltd., Gyeonggi-do 13591 (KR)
(72) Inventor: AHN, Gunyoung, Seongnam-si Gyeonggi-do 13591 (KR)
(74) Representative: Keilitz Haines & Partner Patentanwälte PartGmbB
(86) International application number: PCT/KR2020/014465
(87) International publication number: WO 2022/085817

(57) **Abstract**

The present invention relates to fractional microneedle hybrid switch module manufacturing method, and more particularly, to a fractional microneedle hybrid switch module manufacturing method capable of uniformly distributing high-frequency energy to a portion of the skin and a local site to be treated by partitioning a fractional microneedle patch and applying a high-frequency signal to needles in the partitioned zones, and an electrically isolated switching element is attached to each of partitioned zones so that energy may be efficiently injected, and stable control that can prevent malfunctions due to noise caused by high frequency is possible.

## Description

### [Technical Field]

The present invention relates to a fractional microneedle hybrid switch module manufacturing method, and more particularly, to a fractional microneedle hybrid switch module manufacturing method capable of uniformly distributing high-frequency energy to a portion of the skin and a local site to be treated by partitioning a fractional microneedle patch and applying a high-frequency signal to needles in the partitioned zones.

### [Background Art]

Fractional microneedle technology used in the related art is a fractional high-frequency (radio frequency (RF)) treatment using the physical treatment of microneedles and RF high-frequency energy, and is a treatment that induces scarring and pore relief through collagen regeneration by inserting fractional microneedles into a site in contact with the skin to reduce stimulation on the skin surface and instantaneous discharge high-frequency heat energy to the dermal layer.

Generally, microneedles are used to deliver active substances such as drugs and vaccines in vivo and to detect an analyte in the body and to perform a biopsy. Accordingly, recently, the microneedle is being applied to a treatment device that activates cell tissue by applying high frequency to the microneedle and directly delivering high-frequency energy to the dermal layer of the skin through the microneedle to maintain elastic skin and minimize skin aging. That is, the microneedle is used as a device in which when high-frequency (RF) energy is applied to the human body, the energy is converted into thermal energy in the human body, and the thermal energy raises the temperature inside the human body to obtain a medical treatment or cosmetic effect.

As an example of the microneedle treatment method, Korean Patent Laid-Open Publication No. 2018-0141580 (May 26, 2020) discloses a skin treatment needle configured to increase the speed of skin regeneration by supplying energy into skin tissue, and including a needle body that is formed of a conductive material and inserted into the skin from a front end thereof, wherein the needle body has an outer circumferential surface in which a partial region includes a conductive portion, which is a region inducing an electric field different from those formed at other portions of the needle body, and the conductive portion includes a plurality of creases to cause the electric field formed through the conductive portion to have a uniform distribution without being concentrated in one place, and a skin treatment device.

However, in the case of the fractional microneedle patch of the related art, the high-frequency energy distribution is concentrated only at an edge of the patch into which the needles are inserted, and the energy distribution decreases toward the center portion. That is, there is a problem that the energy distribution is non-uniformly formed and thus the site to be treated is not treated well.

### [Disclosure]

### [Technical Problem]

The present invention is directed to providing a fractional microneedle hybrid switch module manufacturing method, capable of minimizing the damage to the epidermal layer caused by a microneedle to which high frequency is applied, and uniformly distributing energy to each partitioned zone of a patch according to the purpose of treatment so that a portion of the skin and a local site are effectively treated.

### [Technical Solution]

One aspect of the present invention provides a fractional microneedle hybrid switch module manufacturing method including generating a flat plate made of a conductive material, partitioning the flat plate having conductivity into m×m parts to generate N partitioned zones, generating M grooves into which fractional microneedles are inserted in the N partitioned zones and inserting M fractional microneedles into the grooves, connecting each of the M needles inserted into the N partitioned zones with an electrical connection terminal such that a high-frequency signal is applied thereto, and applying high-frequency energy applied to the microneedle to a portion of the skin or a local site using monopolar and bipolar hybrid switch modules.

According to an embodiment of the present invention, the method may include configuring the hybrid switch module including an input needle connected to a switch that is connected to a counter electrode (human body), and an output needle connected to the electrical connection terminal and configured to supply power, and configuring the hybrid switch module in a monopolar or bipolar type depending on a connection type by which the input needle is connected to the counter electrode or the electrical connection terminal.

According to an embodiment of the present invention, the method may include selecting the monopolar type or the bipolar type of the hybrid switch module according to a skin condition of a person and a site to be treated.

According to an embodiment of the present invention, the method may include configuring the hybrid switch module in the monopolar type by connecting the switch connected to the counter electrode to a switch of the input needle and connecting a switch of the electrical connection terminal to the output needle when targeting and healing a portion of the skin of the person, and configuring the hybrid switch module in the bipolar type by connecting the output needle and the switch of the input needle to the switch of the electrical terminal when targeting and healing a local site of the skin of a user is.

According to an embodiment of the present invention, the method may further include forming a ground for each needle itself, to which the hybrid switch module configured in the bipolar type is connected.

### [Advantageous Effects]

In a fractional microneedle hybrid switch module manufacturing method according to the present invention, an electrically isolated switching element is attached to each of partitioned zones so that energy can be efficiently injected, and stable control that can prevent malfunction due to noise caused by high frequency is possible.

### [Description of Drawings]

FIG. 1 is a flowchart of a method of applying energy due to high frequency to a portion of the skin or a local site using monopolar and bipolar hybrid switch modules.
FIGS. 2 and 3 are radio frequency (RF) (ultrasonic) switching circuit diagrams that constitute the monopolar and bipolar switch modules.

### [Modes of the Invention]

A method of manufacturing a needle module using a fractional high frequency according to embodiments of the present invention will be described in detail with reference to the accompanying drawings. While the present invention is susceptible to various modifications and alternative forms, specific embodiments thereof are shown by way of example in the drawings and will herein be described in detail in the text. However, the description is not intended to limit the present invention to the specific embodiments, and it should be understood that the present invention is to cover all modifications, equivalents, and alternatives that fall within the spirit and scope of the present invention. In describing each drawing, like reference numerals represent like components. In the accompanying drawings, dimensions of structures are shown larger than actual dimensions for clarity of the present invention or smaller than actual dimensions in order to understand a schematic configuration.

In addition, while terms such as "first," "second," and the like may be used to describe various components, such components should not be limited to the above terms. These terms are only used to distinguish one component from another. For example, without departing from the scope of the present invention, a first component may be referred to as a second component, and similarly, a second component may be referred to as a first component. Meanwhile, unless otherwise defined, all terms used herein including technical or scientific terms have the same meanings as those generally understood by one of ordinary skill in the art. It should be further understood that terms, such as those defined in commonly used dictionaries, should be interpreted as having a meaning that is consistent with their meaning in the context of the relevant art and are not to be interpreted in an idealized or overly formal sense unless expressly so defined herein.

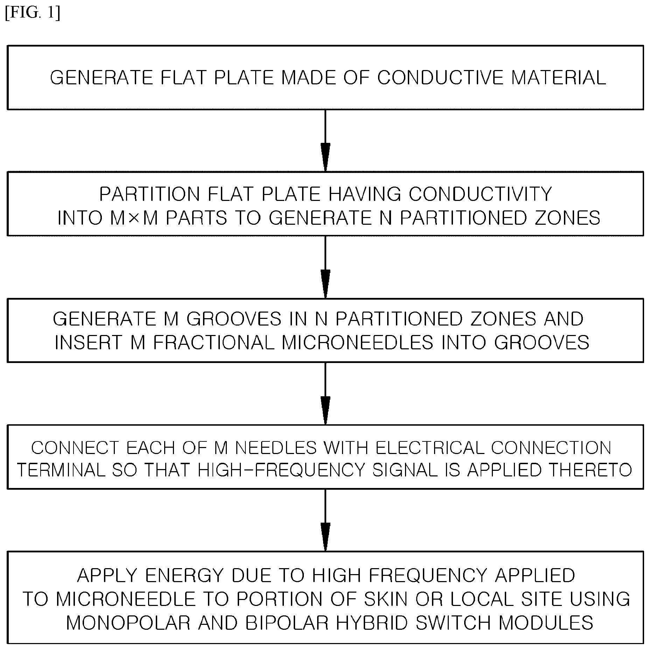

FIG. 1 is a flowchart of a method of applying energy due to high frequency to a portion of the skin and a local site using monopolar and bipolar hybrid switch modules.

Referring to FIG. 1, a fractional microneedle hybrid switch module manufacturing method includes generating a flat plate made of a conductive material, partitioning the flat plate having conductivity into m×m parts to generate N partitioned zones, generating M grooves into which fractional microneedles are inserted in the N partitioned zones and inserting M fractional microneedles into the grooves, connecting each of the M needles inserted into the N partitioned zones with an electrical connection terminal such that a high-frequency signal is applied thereto, and applying high-frequency energy applied to the microneedle to a portion of the skin or a local site using monopolar and bipolar hybrid switch modules.

Here, the method includes configuring a hybrid switch module including an input needle connected to a switch that is connected to a counter electrode (human body), and an output needle connected to the electrical connection terminal and configured to supply power, and configuring the hybrid switch module in a monopolar or bipolar type depending on a connection type by which the input needle is connected to the counter electrode or the electrical connection terminal. The method includes selecting the monopolar type or the bipolar type of the hybrid switch module according to a skin condition of a person and a site to be treated. In addition, the method includes configuring the hybrid switch module in the monopolar type by connecting the switch connected to the counter electrode to a switch of the input needle and connecting a switch of the electrical connection terminal to the output needle when targeting and healing a portion of the skin of the person, and configuring the hybrid switch module in the bipolar type by connecting the output needle and the switch of the input needle to the switch of the electrical connection terminal when targeting and healing a local site of the skin of a user. Here, more specifically, the method further includes forming a ground for each needle itself, to which hybrid switch module configured in the bipolar type is connected.

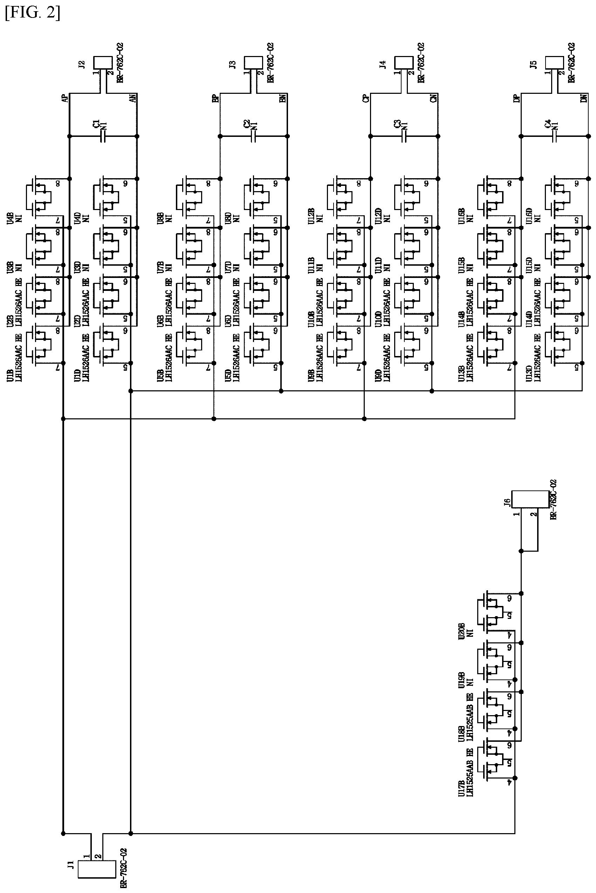

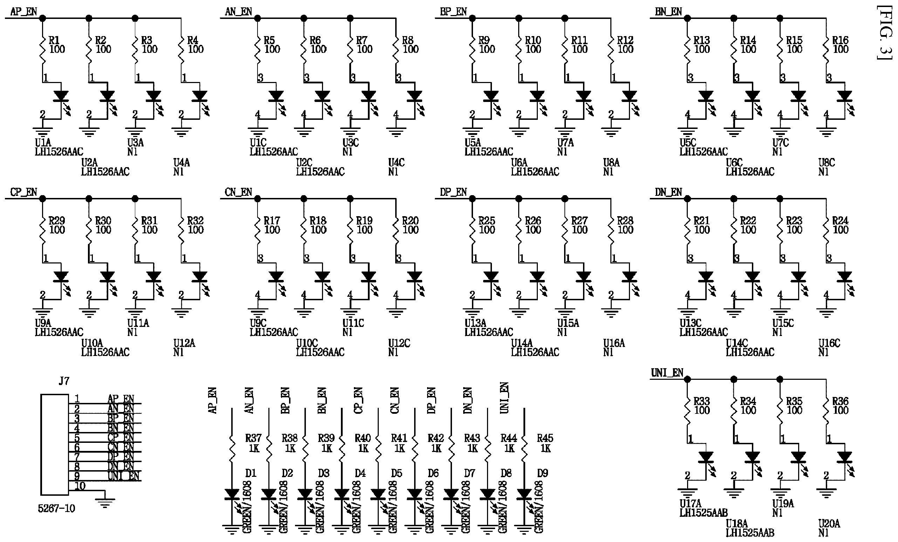

FIGS. 2 and 3 are radio frequency (RF) (ultrasonic) switching circuit diagrams that constitute the monopolar and bipolar switch modules.

Referring to FIGS. 2 and 3, in the present invention, since an electrically isolated switching element is attached to each of partitioned zones, energy may be efficiently injected, and stable control that may prevent malfunctions due to noise caused by high frequency is possible.

Although the exemplary embodiments of the present invention have been described hereinabove, those skilled in the art or those of ordinary skill in the art will understand that the present invention may be variously modified and changed within the scope not departing from the spirit and technical scope of the present invention described in the claims to be described later.

### [Industrial Applicability]

There are various acne scars including minor scars such as acne red marks and acne pigmentation scars that are relatively less visible and can be treated with little disruption to daily life, and severe scars such as pitted scars and hypertrophic scars that take a longer time to treat and require more downtime. In particular, the pitted scars may be classified into ice pick scars having an awl shape, boxcar scars having a box shape, and rolling scars with a rounded recessed shape according to their shape.

Pitted scar treatment began with laser peeling, which was previously treated by peeling off the skin, and took a leap forward with the launch of a laser treatment device called "Fraxel" in the United States in 2004. Currently, skin beauty devices for cosmetic purposes in addition to acne scar treatment have been commercialized. A fractional laser includes a laser having a wavelength such as 1410 nm, 1440 nm, 1540 nm, or 1550 nm which does not cause epidermal damage and a CO2 fractional laser and an Er:YAG fractional laser having a wavelength that may peel off the skin, according to wavelength.

## Claims

1. A fractional microneedle hybrid switch module manufacturing method comprising:
generating a flat plate made of a conductive material;
partitioning the flat plate having conductivity into m×m parts to generate N partitioned zones;
generating M grooves into which fractional microneedles are inserted in the N partitioned zones and inserting M fractional microneedles into the grooves;
connecting each of the M needles inserted into the N partitioned zones with an electrical connection terminal such that a high-frequency signal is applied thereto; and
applying high-frequency energy applied to the microneedle to a portion of the skin or a local site using monopolar and bipolar hybrid switch modules.

2. The method of claim 1, comprising:
configuring the hybrid switch module including an input needle connected to a switch that is connected to a counter electrode (human body), and an output needle connected to the electrical connection terminal and configured to supply power; and
configuring the hybrid switch module in a monopolar or bipolar type depending on a connection type by which the input needle is connected to the counter electrode or the electrical connection terminal.

3. The method of claim 2, comprising selecting the monopolar type or the bipolar type of the hybrid switch module according to a skin condition of a person and a site to be treated.

4. The method of claim 3, comprising configuring the hybrid switch module in the monopolar type by connecting the switch connected to the counter electrode to a switch of the input needle and connecting a switch of the electrical connection terminal to the output needle when targeting and healing a portion of the skin of the person; and
configuring the hybrid switch module in the bipolar type by connecting the output needle and the switch of the input needle to the switch of the electrical connection terminal when targeting and healing a local site of the skin of a user.

5. The method of claim 4, further comprising forming a ground for each needle itself, to which the hybrid switch module configured in the bipolar type is connected.
